# EUROPEAN PATENT APPLICATION

(11) **EP 4 491 183 A1**
(43) Date of publication of application: **15.01.2025**
(21) Application number: 23766134.3
(22) Date of filing: 10.03.2023
(51) Int. Cl.: A61K 31/52, A61K 31/522, A61P 11/00, A61P 31/04, A61P 31/16

(54) **USE OF HYPOXANTHINE COMPOUND IN PREPARATION OF DRUG FOR TREATING PULMONARY FIBROSIS**

(30) Priority: 11.03.2022 CN 202210242716
(71) Applicant: Sichuan Yiasuo Pharmaceutical Technology Co., Ltd., Chengdu, Sichuan 610042 (CN)
(72) Inventor: HUANG, Wen, Chengdu, Sichuan 610041 (CN); LI, Weimin, Chengdu, Sichuan 610041 (CN)
(74) Representative: HGF
(86) International application number: PCT/CN2023/080746
(87) International publication number: WO 2023/169557

(57) **Abstract**

The present invention discloses a use of a hypoxanthine compound in preparing a drug for treating pulmonary fibrosis and relates to the technical field of biological medicines. In the use of the hypoxanthine compound in preparing the drug for treating the pulmonary fibrosis, through pharmacological activity detection for treatment of pulmonary fibrosis with hypoxanthine compounds subjected to structural modification and transformation, the pharmacological activities of such the compounds in various animal disease models are tested and data about the activities of preventing and treating different types of pulmonary fibrosis are provided, which proves that the hypoxanthine compounds have good activities, with an effect being obviously superior to that of a positive drug Nintedanib commonly used in clinic, and that of hypoxanthine analogues A, B, C and D as well as other hypoxanthine analogues in the prior art. The use of the hypoxanthine compound in preparing the drug for treating the pulmonary fibrosis can provide a novel skeleton for screening novel compounds for preparing drugs for treating pulmonary fibrosis and lay a theoretical foundation of developing a novel lead compound.

## Description

### Technical Field

The present invention relates to the technical field of biological medicines, in particular to a use of a hypoxanthine compound in preparing a drug for treating pulmonary fibrosis.

### Background of the Invention

There are various pathogenic factors of pulmonary fibrosis, clinical pathologic manifestations of the pulmonary fibrosis generally include fibroblasts proliferation, aggregation of a large amount of extracellular matrixes, an inflammatory response, destruction of lung tissue structures and the like. The fatality rate of the pulmonary fibrosis is very high and even higher than that of most tumors; and an average survival period is 2.8 years after the pulmonary fibrosis is confirmed. Pneumonia progresses and eventually develops pulmonary fibrosis; they are different manifestations of the same type of disease in different development stages; and deterioration of interstitial pneumonia or other types of pneumonia is characterized by pulmonary fibrosis. Therefore, finding a drug capable of preventing and treating pneumonia and pulmonary fibrosis, alleviating lung inflammatory responses, and blocking mild pneumonia from being transformed into pulmonary fibrosis as severe pneumonia is crucial for preventing and treating pneumonia and pulmonary fibrosis.

Hypoxanthine, also referred to as "6-hypoxanthine", is a naturally occurring purine compound, which is a synthetic precursor for purine nucleotides of nucleic acids. At present, there are no reports on hypoxanthine compounds capable of blocking development to pulmonary fibrosis and reversing pathological injuries.

### Summary of the Invention

An objective of the present invention is to provide a use of a hypoxanthine compound in preparing a drug for treating pulmonary fibrosis, so as to accelerate the research and development process of a novel drug for treating pulmonary fibrosis. Many technical effects that can be produced by a preferred technical solution of the present invention are described in detail hereinafter.

In order to achieve the above objective, the present invention provides the following technical solution:
The first aspect of the present invention relates to a use of a hypoxanthine compound in preparing a drug for treating pulmonary fibrosis, wherein the hypoxanthine compound has the activity of treating the pulmonary fibrosis, and the hypoxanthine compound has one of the following structures: 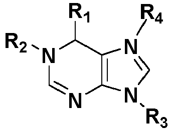 wherein:
R₁ is optionally O, N, C, S or =O;
R₂, R₃ and R₄ are optionally H, C₁-C₁₈ alkyl, C₁-C₁₈ alkyl substituted with a halogen, trifluoromethyl, sulfonyl, sulfonamide, sulfinyl, an amino acid group, 2-[bis[(pivaloyloxy)methoxy]phosphinyl]methoxy]ethyl, a C₁-C₁₈ fatty acid group, C₃-C₁₂ heterocyclyl, and C₁-C₁₈ fatty acid; or R₂, R₃ and R₄ are optionally C₁-C₁₈ alkyl or fatty acid group substituted with an oxygen atom, a sulphur atom or a nitrogen atom; and R₃ and R₄ are attached to a non-substituted position N via double bonds when being monosubstituted and have no double bonds when being fully substituted.

The second aspect of the present invention relates to a compound having the following structure: 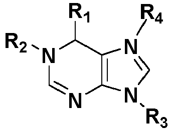 wherein:
R₁ is optionally O, N, C, S or =O;
R₂, R₃ and R₄ are optionally H, C₁-C₁₈ alkyl, C₁-C₁₈ alkyl substituted with a halogen, a trifluoromethyl, sulfonyl, sulfonamide, sulfinyl, an amino acid group, 2-[bis[(pivaloyloxy)methoxy]phosphinyl]methoxy]ethyl, a C₁-C₁₈ fatty acid group, C₃-C₁₂ heterocyclyl, and C₁-C₁₈ fatty acid; or R₂, R₃ and R₄ are optionally C₁-C₁₈ alkyl or fatty acid group substituted with an oxygen atom, a sulphur atom or a nitrogen atom; and R₃ and R₄ are attached to a non-substituted position N via double bonds when being monosubstituted and have no double bonds when being fully substituted.

According to one preferred embodiment, the compound is selected from the following group:

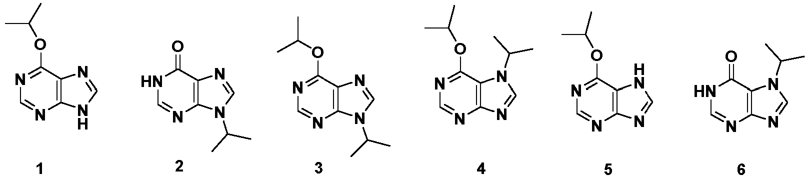

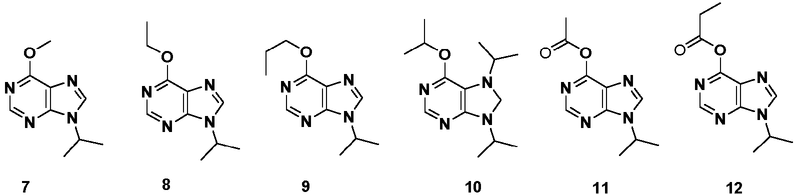

The third aspect of the present invention relates to a pharmaceutical composition comprising the compound of the present invention or the pharmaceutically acceptable salt thereof.

According to one preferred embodiment, the pharmaceutical composition further comprises the pharmaceutically acceptable excipient or auxiliary component.

According to one preferred embodiment, the pharmaceutical composition is an oral preparation, an injection preparation or a nasal mucosal administration preparation.

The fourth aspect of the present invention relates to a method of treating pulmonary fibrosis, the method comprising: administrating an effective amount of compound or the pharmaceutically acceptable salts thereof, or the pharmaceutical composition of the present invention to a subject in need thereof.

According to one preferred embodiment, the drug for treating the pulmonary fibrosis comprises a drug having the efficacy of preventing and treating the pulmonary fibrosis and complications thereof.

According to one preferred embodiment, the drug for treating the pulmonary fibrosis is a preparation prepared by using the hypoxanthine compound or a salt thereof as an active ingredient in combination with pharmaceutically acceptable excipient or auxiliary component.

According to one preferred embodiment, the preparation is an oral preparation, an injection preparation or a nasal mucosal administration preparation.

According to one preferred embodiment, the pulmonary fibrosis comprises one or more of primary pulmonary fibrosis, secondary pulmonary fibrosis, idiopathic pulmonary fibrosis, pulmonary interstitial fibrosis, and interstitial pneumonia.

According to one preferred embodiment, the pulmonary fibrosis comprises one or more of bacterial pulmonary fibrosis, viral pulmonary fibrosis, mycoplasma pulmonary fibrosis, chlamydia pulmonary fibrosis, immunological pulmonary fibrosis, and fungus pulmonary fibrosis. According to one preferred embodiment, the pulmonary fibrosis comprises one or more of *Streptococcus pneumoniae-induced* pulmonary fibrosis, influenza A virus-induced pulmonary fibrosis, influenza B virus-induced pulmonary fibrosis, coronavirus-induced pulmonary fibrosis, and novel coronavirus-induced pulmonary fibrosis.

According to one preferred embodiment, the pulmonary fibrosis further comprises one or more of *Klebsiella pneumonia*-induced pulmonary fibrosis, *Streptococcus pneumoniae-induced* pulmonary fibrosis, vancomycin-resistant *Enterococcus*-induced pulmonary fibrosis, drug-resistant *Staphylococcus aureus*-induced pulmonary fibrosis, and *Acinetobacter baumannii*-induced pulmonary fibrosis.

### Term definition:

The compound and derivatives thereof provided by the present invention can be named according to a naming system of International Union of Pure and Applied Chemistry (IUPAC) or Chemical Abstracts Service (CAS, Columbus, OH).

The term "alkyl" refers to a straight or branched chain saturated hydrocarbyl group. An example of C₁-C₃ alkyl includes methyl (C₁), ethyl g(C₂), n-propyl (C₃), and isopropyl (C₃).

The term "pharmaceutically acceptable" refers to that a certain carrier, a vehicle, a diluent, an accessory and/or a formed salt is generally chemically or physically compatible with other components forming a certain drug dosage form, and is physiologically compatible with a receptor.

The term "pharmaceutically acceptable salt" refers to an acidic and/or basic salt formed by the compound of the present invention and inorganic and/or organic acids and bases, also including a zwitterionic salt (an inner salt) and further including a quaternary ammonium salt, for example, an alkyl ammonium salt. These salts can be obtained directly in final separation and purification of the compound; and can also be obtained by properly mixing the above compound with a certain quantity (for example, equal equivalence) of acids or bases. These salts may be collected by forming precipitates in a solution and filtering the precipitates, or recovered after evaporation of a solvent, or prepared by freezing and drying after reaction in an aqueous medium. The salts in the present invention can be hydrochloride, sulfate, citrate, benzene sulfonate, hydrobromide, hydrofluoride, phosphate, acetate, propionate, succinate, oxalate, malate, succinate, fumarate, maleate, tartrate or trifluoroacetate of the compound.

There is no specific limitation to administration modes for the compound or pharmaceutical composition of the present invention; and representative administration modes include (but are not limited to): oral administration, parenteral (intravenous, intramuscular and subcutaneous) administration and local administration.

Solid dosage forms for oral administration include capsules, tablets, pills, powder and particles. In these solid dosage forms, active compounds are mixed with at least one conventional inert excipient (or a carrier) such as sodium citrate or dicalcium phosphate, or mixed with the following components: (a) a filler or a solubilizer, for example, starch, lactose, sucrose, glucose, mannitol and silicic acid; (b), an adhesive, for example, hydroxymethyl cellulose, alginate, gelatin, polyvinyl pyrrolidone, sucrose and gum Arabic; (c) a humectant, for example, glycerol; (d) a disintegrating agent, for example, agar, calcium carbonate, potato starch or tapioca starch, alginic acid, certain compound silicate, and sodium carbonate; (e) a retarding solvent, for example, paraffin; (f) an absorption accelerator, for example, a quaternary ammonium compound; (g) a wetting agent, for example, cetyl alcohol and glyceryl monostearate; (h) an adsorbent, for example, kaolin; and (i) a lubricant, for example, talc, calcium stearate, magnesium stearate, solid polyethylene glycol, lauryl sodium sulfate or a mixture thereof. In the case of the capsules, the tablets and the pills, the dosage form can further include a buffer agent.

The solid dosage forms such as tablets, sugar pills, capsules, pills and particles can be prepared by using coatings and shell materials such as casings and other materials well known in the art. The solid dosage forms can contain opacifying agents, and active compounds or compounds in such compositions can be released in a certain part of a digestive tract in a manner of delaying. Examples of embedded components that can be used are a polymeric substance and a wax substance. If necessary, the active compounds together with one or more of the above excipients can further form microcapsules.

Liquid dosage forms for oral administration include pharmaceutically acceptable emulsions, solutions, suspensions, syrups or tinctures. In addition to the active compounds, the liquid dosage forms can contain conventionally used inert diluents such as water or other solvents, a solubilizer and an emulsifier, for example, ethyl alcohol, isopropanol, ethyl carbonate, ethyl acetate, propylene glycol, 1,3-butanediol, dimethylformamide or oil, particularly cottonseed oil, peanut oil, maize germ, olive oil, castor oil, sesame oil or a mixture thereof.

In addition to these inert diluents, the composition can further comprise an adjuvant such as a wetting agent, an emulsifier, a suspending agent, a sweetening agent, a flavoring agent and a flavor.

In addition to the active compounds, the suspension can comprise a suspending agent, for example, ethoxylated isostearyl alcohols, polyoxyethylene sorbitol and sorbitan ester, microcrystalline cellulose, aluminum methoxide and agar or a mixture thereof.

Compositions for parenteral injection can comprise physiologically acceptable sterile aqueous or anhydrous solutions, dispersions, suspensions or emulsions, and sterile powders for redissolution into sterile injectable solutions or dispersions. Suitable aqueous or nonaqueous carriers, diluents, solvents or excipients include water, ethyl alcohol, polyhydric alcohols and a suitable mixture thereof.

Dosage forms of a compound for topical administration of the present invention include ointments, powders, patches, sprayers and inhalants, for example, nasal mucosal administration preparations. The active component is admixed, under sterile conditions, with a physiologically acceptable carrier, and any preservative and buffer agent, or any propellant that may be required if necessary. The pharmaceutically acceptable accessories of the present invention refer to other substances included in the dosage forms in addition to the active components.

The pharmaceutically acceptable auxiliary component of the present invention has certain physiological activities. However, with the addition of such the auxiliary component, the leading role of the above pharmaceutical composition in the disease treatment process cannot be changed, while the auxiliary component only exerts auxiliary efficacies. The auxiliary efficacies are generated only by utilization of the known activities of the auxiliary component, and therefore the addition of such the auxiliary component is a conventional auxiliary treatment manner in the field of medicines. If the above auxiliary component is combined with the pharmaceutical composition of the present invention, they should still fall within the scope of protection of the present invention.

The hypoxanthine compound provided by the present invention at least has the following beneficial effects:
In the use of the hypoxanthine compound in preparing the drug for resisting the pulmonary fibrosis provided by the present invention, through pharmacological activity detection for treatment of pulmonary fibrosis with the hypoxanthine compounds subjected to structural modification and transformation, the pharmacological activities of such the compounds in various animal disease models are tested and about the activities of preventing and treating different types of pulmonary fibrosis are provided, which proves that the hypoxanthine compounds all have good activities, with an effect being obviously superior to that of positive drug Nintedanib commonly used in clinic, and that of analogues A, B, C and D as well as other hypoxanthine analogues in the prior art. That is, the use of the hypoxanthine compound in preparing the drug for resisting the pulmonary fibrosis provided by the present invention can provide a novel skeleton for screening novel compounds for preparing drugs for resisting pulmonary fibrosis and lay a theoretical foundation of developing a novel lead compound.

Specifically, the use of the hypoxanthine compound in preparing the drug for resisting the pulmonary fibrosis provided by the present invention can significantly improve the pulmonary fibrosis caused by viruses and bacteria. More importantly, the hypoxanthine compound provided by the present invention can improve the symptoms of early pulmonary fibrosis and the reverse symptoms of advanced pulmonary fibrosis.

### Detailed Description of the Invention

In order to make the objectives, technical solutions and advantages of the present invention clearer, the technical solutions of the present invention will be described in detail below. Apparently, the described embodiments are merely some embodiments, of the present invention, but not all the embodiments. Based on the embodiments of the present invention, all other embodiments including extended researches on the hypoxanthine compound of the present invention in treatment of pulmonary fibrosis made by a person of ordinary skill in the art without creative efforts are included within the scope of protection of the present invention.

Through pharmaceutical chemical and chemical researches on natural products, many plant endogenous compounds and derivatives have been developed. By modifying and transforming the structures of the natural products, many derivatives having excellent pharmacological activities are obtained. In the use of the hypoxanthine compound in preparing the drug for resisting the pulmonary fibrosis provided by the present invention, through pharmacological activity detection for treatment of pulmonary fibrosis with of the hypoxanthine compounds subjected to structural modification and transformation, the pharmacological activities of such the compounds in various animal disease models are tested and data about the activities of preventing and treating different types of pulmonary fibrosis are provided, which proves that the hypoxanthine compounds have good activities, with an effect being obviously superior to that of positive drug Nintedanib commonly used in clinic, and that of hypoxanthine analogues A, B, C and D as well as other hypoxanthine analogues in the prior art. That is, the use of the hypoxanthine compound in preparing the drug for resisting the pulmonary fibrosis provided by the present invention can provide a novel skeleton for screening novel compounds for preparing the drugs for resisting the pulmonary fibrosis and lay a theoretical foundation of developing a novel lead compound.

Structures of the hypoxanthine analogues A, B, C and D are respectively shown as follows:

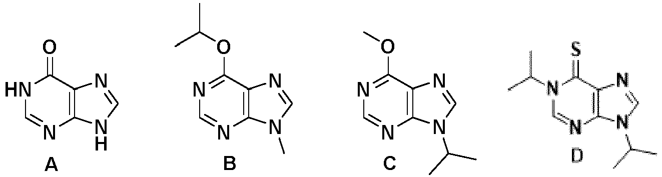

The hypoxanthine analogues A, B, C and D are prepared by the following methods for preparing compound 2, compound 4 and/or compound 6.

Next, the use of the hypoxanthine compound in preparing the drug for resisting the pulmonary fibrosis will be described in detail in combination with examples 1-8.

### Example 1

### Preparation methods of compounds 1-12 are described in detail in this example.

According to a preferred embodiment, the compounds 1-12 are prepared by alkylation reaction of hypoxanthine.

This example provides preparation methods of 12 compounds. The structures of the obtained compounds are all determined via nuclear magnetic resonance spectroscopy and mass spectroscopy.

### Preparation of compound 1 and compound 5

Synthetic routes of compound 1 and compound 5 are shown as follows:

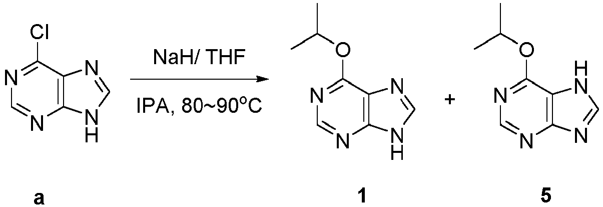

Reaction: (1) 3.45 g of NaH (4.5 eq) was added in a 250 mL four-necked bottle at 0°C, and after air was sucked and exchanged for 3 times, 40 mL of (8 V) anhydrous THF was slowly added at N₂ atmosphere; (2) 12.3 mL of isopropanol (4.5 eq) was slowly added to a system dropwise to react for 30 min; (3) then, a mixture of 5 g of compound 1 (1.0 eq) in 100 mL of (20 V) isopropanol was slowly added to a reaction system dropwise; and (4) a temperature was raised to 80°C, and the system reacted for 10 h.

Posttreatment: (1) after the reaction was completed, a resultant was quenched with water, and acetic acid was added for neutralizing until a pH value was equal to 8-10; (2) the resultant was extracted with ethyl acetate for 5 times, and organic phases were combined; (3) the combined organic phase was dried with anhydrous sodium sulfate, concentrated and rapidly separated by silica gel column chromatography to obtain a light yellow solid; and (4) thin layer chromatography (TLC) monitoring: a developing solvent: dichloromethane/methyl alcohol = 10: 1Rf (compound 1) = 0.4.

Relevant spectral data of compound 1 and compound 5 is as follows:
Compound **1:** 1H NMR (400 MHz, DMSO-d6) δ 13.33 (s, 1H), 8.44 (s, 1H), 8.32 (s, 1H), 5.55 (hept, J = 6.1 Hz, 1H), 1.37 (d, J = 6.2 Hz, 6H). HRMS (ESI-TOF) calc'd for C8H10N4OH+ [M + H+]: 179.09; found 179.10.
Compound **5:** 11H NMR (400 MHz, DMSO-d6) δ 13.37 (s, 1H), 8.47 (s, 1H), 8.35 (s, 1H), 5.57 (hept, J = 6.2 Hz, 1H), 1.39 (d, J = 6.2 Hz, 6H). HRMS (ESI-TOF) [M + H+]: 179.09; found 179.00.

### Preparation of compound 2, compound 4 and compound 6

Synthetic routes of compound 2, compound 4 and compound 6 are shown as follows:

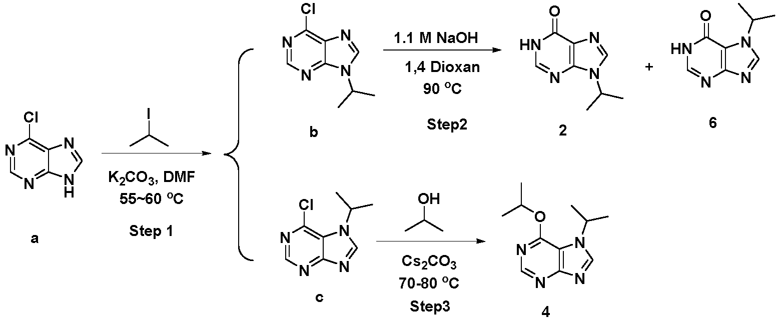

Relevant spectral data of compound 2, compound 4 and compound 6 is as follows:
Compound 2: ¹H NMR (400 MHz, Chloroform-d) δ 13.19 (s, 1H), 8.24 (s, 1H), 7.89 (s, 1H), 4.80 (hept, J = 6.8 Hz, 1H), 1.60 (d, J = 6.8 Hz, 6H). HRMS (ESI-TOF) [M + H+]: 179.19; found 179.30.
Compound **4:** ¹H NMR (400 MHz, Chloroform-d) δ 8.58 (s, 1H), 8.07 (s, 1H), 5.63 (hept, J = 6.2 Hz, 1H), 4.90 (hept, J = 6.7 Hz, 1H), 1.59 (d, J = 6.8 Hz, 6H), 1.44 (d, J = 6.2 Hz, 6H). HRMS (ESI-TOF) [M + H+]: 221.27; found 221.20.
Compound **6:** ¹H NMR (400 MHz, Chloroform-d) δ 13.21 (s, 1H), 8.27 (s, 1H), 7.82 (s, 1H), 4.70 (hept, J = 6.8 Hz, 1H), 1.65 (d, J = 6.6 Hz, 6H). HRMS (ESI-TOF) [M + H+]: 179.19; found 179.30.

### Preparation of compound 3

A synthetic route of compound 3 is shown as follows:

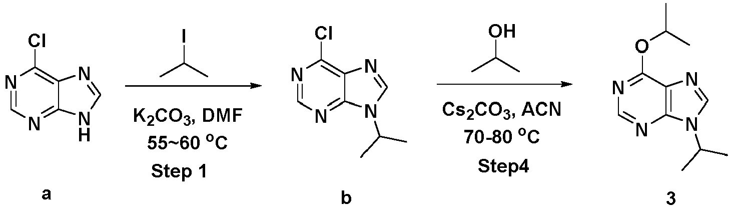

Relevant spectral data of compound 3 is as follows:
Compound **3:** ¹H NMR (400 MHz, DMSO-d6) δ 8.43 (s, 1H), 8.19 (s, 1H), 5.08 (hept, J = 6.9 Hz, 1H), 4.71 (hept, J = 6.8 Hz, 1H), 1.52 (d, J = 6.8 Hz, 6H), 1.41 (d, J = 6.9 Hz, 6H). HRMS (ESI-TOF) [M + H+]: 221.27; found 221.30.

### Preparation of compounds 7-12

Compounds 7-12 are prepared by the above method of preparing one of compounds 1-6. Relevant spectral data of compounds 7-12 is as follows:
Compound **7:** ¹H NMR (500 MHz, Chloroform-d) δ 8.58 (s, 1H), 7.99 (d, J = 0.9 Hz, 1H), 4.89 (heptd, J = 5.6, 0.5 Hz, 1H), 4.18 (s, 3H), 1.63 (s, 6H). HRMS (ESI-TOF) [M + H+]: 193.10; found 193.10.
Compound **8:** ¹H NMR (500 MHz, Chloroform-d) δ 8.55 (s, 1H), 7.95 (d, J = 0.9 Hz, 1H), 4.85 (heptd, J = 5.2, 0.5 Hz, 1H), 4.62 (q, J = 6.5 Hz, 2H), 1.57 (s, 3H), 1.50 (t, J = 6.5 Hz, 3H). HRMS (ESI-TOF) [M + H+]: 207.12; found 207.10.
Compound **9:** ¹H NMR (500 MHz, Chloroform-d) δ 8.51 (s, 1H), 7.57 (d, J = 0.3 Hz, 1H), 4.57 (heptd, J = 5.0, 0.7 Hz, 1H), 4.61 (t, J = 5.4 Hz, 2H), 1.90 (qt, J = 7.3, 5.9 Hz, 2H), 1.63 (s, 6H), 1.15 (t, J = 7.4 Hz, 3H). HRMS (ESI-TOF) [M + H+]: 221.13; found 221.10.
Compound **10:** ¹H NMR (500 MHz, Chloroform-d) δ 8.37 (s, 1H), 5.33 - 5.27 (m, 3H), 4.74 - 4.64 (m, 2H), 1.25 (d, J = 6.2 Hz, 6H), 1.17 (dd, J = 6.7, 5.2 Hz, 12H). HRMS (ESI-TOF) [M + H+]: 265.20; found 265.20.
Compound **11:** ¹H NMR (500 MHz, Chloroform-d) δ 7.99 - 7.88 (m, 2H), 4.83 (pd, J = 5.6, 0.8 Hz, 1H), 2.32 (s, 3H), 1.53 (s, 6H). HRMS (ESI-TOF) [M + H+]: 221.10; found 221.10.
Compound **12:** ¹H NMR (500 MHz, Chloroform-d) δ 7.99 - 7.90 (m, 2H), 4.85 (heptd, J = 5.2, 0.5 Hz, 1H), 2.54 (q, J = 7.7 Hz, 2H), 1.55 (s, 3H), 1.19 - 1.11 (m, 3H). HRMS (ESI-TOF) [M + H+]: 235.10; found 235.10.

### Example 2

### This example is used for detecting the extensive pulmonary fibrosis resisting activities of compounds 1-12 prepared in example 1.

### (1) Early pulmonary fibrosis resisting activities of compounds 1-12.

Experimental method: an in-vivo bleomycin-induced pulmonary fibrosis model was established: SPF class C57BL/6 mice (with weights being 22-25 g) were randomly and evenly divided into a plurality of groups, including blank control group, model group, Nintedanib positive control group, hypoxanthine analogue A group, hypoxanthine analogue B group, hypoxanthine analogue C group, compound 1 group, compound 2 group......compound 12 group, with 9 mice in each group, and were fed for 7 days in an SPF class animal center. The mice fasted for 12 h the day before an experiment, then were anesthetized with pentobarbital sodium, inhaled bleomycin (5 mg/kg) to lungs through tracheas by spontaneous breathing to induce pulmonary fibrosis; in addition to that the mice in blank control group received sterile PBS, the mice in model group, Nintedanib positive control group, hypoxanthine analogue A group, hypoxanthine analogue B group, hypoxanthine analogue C group, compound 1 group, compound 2 group......compound 12 group received an equal volume of 5 mg/kg bleomycin. After the model was established for one week, the mice in each group were intragastrically administrated for intervention; the mice in blank control group and model group received the same weight and volume of normal saline as those in drug groups according to the weights; the mice in Nintedanib positive control group received a suitable volume of Nintedanib (120 mg/kg) according to the weights; the mice in hypoxanthine analogue A group, hypoxanthine analogue B group and hypoxanthine analogue C group respectively received hypoxanthine analogue A (120 mg/kg/d), hypoxanthine analogue B (120 mg/kg/d) and hypoxanthine analogue C (120 mg/kg/d); and the mice in compound 1 group, compound 2 group......compound 12 group respectively received compound 1 (120 mg/kg/d), compound 2 (120 mg/kg/d)......compound 12 (120 mg/kg/d) prepared in example 1. The mice were administrated twice every day; after administration for 14 days, the mice were anesthetized with a 0.8% pentobarbital sodium solution (10 mL/kg) on Day 15; blood was taken from aorta abdominalis for detecting changes on blood routine and evaluating the severity of the pulmonary fibrosis of lung tissues; the paraffin sections of the lung tissues were subjected to HE staining and Ashcroft scoring. Results are shown in Table 2-1 and Table 2-2.

**Table 2-1 Table for inflammatory detection results of early pulmonary fibrosis**

| Group | Leucocyte (10⁹/L) | Neutrophil (10⁹/L) | Lymphocyte (10⁹/L) |
|---|---|---|---|
| Blank control group | 4.06 | 0.98 | 5.06 |
| Model group | 67.2 | 40.67 | 53.39 |
| Nintedanib positive control group | 33.76 | 31.32 | 33.87 |
| Hypoxanthine analogue A group | 52.12 | 41.21 | 49.53 |
| Hypoxanthine analogue B group | 49.97 | 36.88 | 50.61 |
| Hypoxanthine analogue C group | 50.11 | 37.04 | 50.06 |
| Compound 1 | 4.15 | 1.11 | 5.42 |
| Compound 2 | 4.11 | 1.04 | 5.19 |
| Compound 3 | 4.01 | 0.96 | 4.99 |
| Compound 4 | 4.29 | 1.31 | 5.56 |
| Compound 5 | 4.23 | 1.26 | 5.60 |
| Compound 6 | 4.25 | 1.19 | 5.49 |
| Compound 7 | 4.19 | 1.23 | 5.44 |
| Compound 8 | 4.27 | 1.26 | 5.31 |
| Compound 9 | 4.14 | 1.33 | 5.23 |
| Compound 10 | 4.17 | 1.21 | 5.27 |
| Compound 11 | 4.09 | 1.10 | 5.20 |
| Compound 12 | 4.21 | 1.18 | 5.24 |

**Table 2-2 Ashcroft scoring table for extent of early pulmonary fibrosis**

| Group | Ashcroft scores |
|---|---|
| Blank control group | 1.03 |
| Model group | 6.53 |
| Nintedanib positive control group | 3.69 |
| Hypoxanthine analogue A group | 5.00 |
| Hypoxanthine analogue B group | 5.39 |
| Hypoxanthine analogue C group | 5.46 |
| Compound 1 | 0.99 |
| Compound 2 | 1.01 |
| Compound 3 | 0.92 |
| Compound 4 | 1.18 |
| Compound 5 | 1.22 |
| Compound 6 | 1.08 |
| Compound 7 | 1.11 |
| Compound 8 | 1.09 |
| Compound 9 | 1.12 |
| Compound 10 | 1.17 |
| Compound 11 | 1.08 |
| Compound 12 | 1.14 |

From data in Table 2-1, it can be seen that the compounds 1-12 prepared in example 1 have a significant inhibition effect on leucocytes, neutrophils and lymphocytes in blood of mice with early pulmonary fibrosis; while the anti-inflammatory effects of Nintedanib tablets, the hypoxanthine analogue A, the hypoxanthine analogue B and the hypoxanthine analogue C are obviously inferior to those of the compounds 1-12.

From data in Table 2-2, it can be obviously seen that the extent of the pulmonary fibrosis of pulmonary fibrosis model mice is significantly improved under the actions of the compounds 1-12 prepared in example 1, which shows that the compounds 1-12 prepared in example 1 have significant pulmonary fibrosis resisting effects, and are superior to the Nintedanib tablets, the hypoxanthine analogue A, the hypoxanthine analogue B and the hypoxanthine analogue C in improvement effect.

### (2) Advanced pulmonary fibrosis resisting activities of compounds 1-12.

Experimental method: an in-vivo bleomycin-induced pulmonary fibrosis model was established: SPF class C57BL/6 mice (with weights being 22-25 g) were randomly and evenly divided into a plurality of groups, including blank control group, model group, Nintedanib positive control group, hypoxanthine analogue A group, hypoxanthine analogue B group, hypoxanthine analogue C group, compound 1 group, compound 2 group......compound 12 group, with 9 mice in each group, and were fed for 7 days in an SPF class animal center. The mice fasted for 12 h the day before an experiment, then were anesthetized with pentobarbital sodium, inhaled bleomycin (5 mg/kg) to lungs through tracheas by spontaneous breathing to induce pulmonary fibrosis; in addition to that the mice in blank control group received sterile PBS, the mice in model group, Nintedanib positive control group, hypoxanthine analogue A group, hypoxanthine analogue B group, hypoxanthine analogue C group, compound 1 group, compound 2 group......compound 12 group received an equal volume of 5 mg/kg bleomycin. After the model was established for three weeks, the mice in each group were intragastrically administrated for intervention; the mice in blank control group and model group received the same weight and volume of normal saline as those in drug groups according to the weights; the mice in Nintedanib positive control group received a suitable volume of Nintedanib (120 mg/kg) according to the weights; the mice in hypoxanthine analogue A group, hypoxanthine analogue B group and hypoxanthine analogue C group respectively received hypoxanthine analogue A (120 mg/kg/d), hypoxanthine analogue B (120 mg/kg/d) and hypoxanthine analogue C (120 mg/kg/d); and the mice in compound 3 group, compound 1 group, compound 2 group......compound 12 group respectively received compound 1 (120 mg/kg/d), compound 2 (120 mg/kg/d)......compound 12 (120 mg/kg/d) prepared in example 1. The mice were administrated for twice every day; after administration for 14 days, the mice were anesthetized with a 0.8% pentobarbital sodium solution (10 mL/kg) on Day 15; blood was taken from aorta abdominalis for detecting changes on blood routine and evaluating severity of the pulmonary fibrosis of lung tissues; the paraffin sections of the lung tissues were subjected to HE staining and Ashcroft scoring. Results are shown in Table 2-3 and Table 2-4.

**Table 2-3 Table for inflammatory detection results of advanced pulmonary fibrosis**

| Group | Leucocyte (10⁹/L) | Neutrophil (10⁹/L) | Lymphocyte (10⁹/L) |
|---|---|---|---|
| Blank control group | 4.36 | 1.29 | 5.55 |
| Model group | 73.67 | 66.77 | 76.98 |
| Nintedanib positive control group | 46.09 | 40.99 | 50.03 |
| Hypoxanthine analogue A group | 66.48 | 67.48 | 71.65 |
| Hypoxanthine analogue B group | 71.35 | 68.34 | 67.37 |
| Hypoxanthine analogue C group | 72.55 | 59.66 | 70.88 |
| Compound 1 | 4.28 | 1.37 | 5.33 |
| Compound 2 | 4.16 | 1.26 | 5.29 |
| Compound 3 | 3.98 | 1.19 | 5.18 |
| Compound 4 | 4.26 | 1.34 | 5.63 |
| Compound 5 | 4.34 | 1.36 | 5.74 |
| Compound 6 | 4.58 | 1.59 | 5.94 |
| Compound 7 | 4.61 | 1.44 | 5.65 |
| Compound 8 | 4.59 | 1.42 | 5.73 |
| Compound 9 | 4.69 | 1.46 | 5.69 |
| Compound 10 | 4.26 | 1.38 | 5.71 |
| Compound 11 | 4.22 | 1.39 | 5.57 |
| Compound 12 | 4.04 | 1.23 | 5.65 |

**Table 2-4 Ashcroft scoring table for extent of advanced pulmonary fibrosis**

| Group | Ashcroft scores |
|---|---|
| Blank control group | 1.08 |
| Model group | 6.69 |
| Nintedanib positive control group | 4.13 |
| Hypoxanthine analogue A group | 5.88 |
| Hypoxanthine analogue B group | 6.01 |
| Hypoxanthine analogue C group | 6.06 |
| Compound 1 | 0.95 |
| Compound 2 | 0.88 |
| Compound 3 | 0.87 |
| Compound 4 | 1.24 |
| Compound 5 | 1.22 |
| Compound 6 | 1.19 |
| Compound 7 | 1.13 |
| Compound 8 | 1.21 |
| Compound 9 | 1.11 |
| Compound 10 | 1.16 |
| Compound 11 | 1.07 |
| Compound 12 | 1.13 |

From Table 2-3, it can be seen that the compounds 1-12 prepared in example 1 have significant inhibition effects on leucocytes, neutrophils and lymphocytes in blood of mice with advanced pulmonary fibrosis; while the anti-inflammatory effects of the Nintedanib tablets, the hypoxanthine analogue A, the hypoxanthine analogue B and the hypoxanthine analogue C are obviously inferior to those of the compounds 1-12.

From Table 2-4, it can be obviously seen that the extent of the pulmonary fibrosis of the pulmonary fibrosis model mice is significantly improved under the actions of the compounds 1-12 prepared in example 1, which shows that the compounds 1-12 prepared in example 1 have the significant pulmonary fibrosis resisting effect, and are superior to the Nintedanib tablets, the hypoxanthine analogue A, the hypoxanthine analogue B and the hypoxanthine analogue C in improvement effect.

### (3) Comparison between pulmonary fibrosis resisting activities of compounds 1-12 and comparative drugs

The structures of hypoxanthine analogues A, B, C and D, as comparative drugs, and compounds E1-E12 are shown as follows:

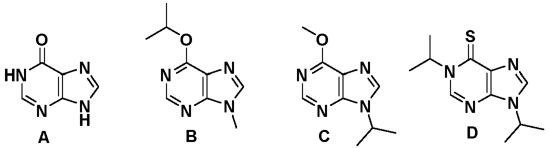

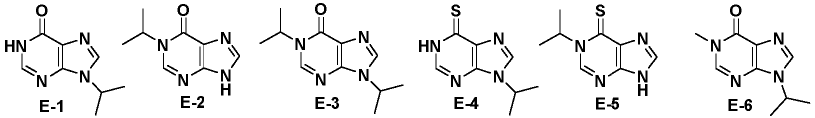

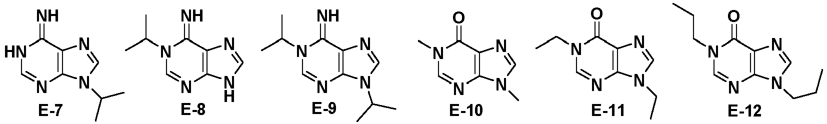

The hypoxanthine analogues A, B, C and D and compounds E1-E12 are prepared by the following methods for preparing compound 2, compound 4 and/or compound 6.

Experimental method: an in-vivo bleomycin-induced pulmonary fibrosis model was established: SPF class C57BL/6 mice (with weights being 22-25 g) were randomly and evenly divided into a plurality of groups, including blank control group, model group, Nintedanib positive control group, hypoxanthine analogue A group, hypoxanthine analogue B group, hypoxanthine analogue C group, hypoxanthine analogue D group, compound E1-E12 groups, compound 1 group, compound 2 group......compound 12 group, with 9 mice in each group, and were fed for 7 days in an SPF class animal center. The mice fasted for 12 h the day before an experiment, then were anesthetized with pentobarbital sodium, inhaled bleomycin (5 mg/kg) to lungs through tracheas by using mouse spontaneous breathing to induce pulmonary fibrosis; in addition to that the mice in blank control group received sterile PBS, the mice in model group, Nintedanib positive control group, hypoxanthine analogue A group, hypoxanthine analogue B group, hypoxanthine analogue C group, hypoxanthine analogue D group, compound E1-E12 groups, compound 1 group, compound 2 group......compound 12 group received an equal volume of 5 mg/kg bleomycin. After the model was established for two weeks, the mice in each group were intragastrically administrated for intervention; the mice in blank control group and model group received the same weight and volume of normal saline as those in drug groups according to the weights; the mice in Nintedanib positive control group received a suitable volume of Nintedanib (120 mg/kg) according to the weights; the mice in hypoxanthine analogue A group, hypoxanthine analogue B group, hypoxanthine analogue C group and hypoxanthine analogue D group respectively received hypoxanthine analogue A (120 mg/kg/d), hypoxanthine analogue B (120 mg/kg/d), hypoxanthine analogue C (120 mg/kg/d) and hypoxanthine analogue D (120 mg/kg/d); the mice in compound 1 group, compound 2 group......compound 12 group respectively received compound 1 (120 mg/kg/d), compound 2 (120 mg/kg/d)......compound 12 (120 mg/kg/d) prepared in example 1; and the mice in compound E1 group, compound E2 group......compound E12 group respectively received compound E1 (120 mg/kg/d), compound E2 (120 mg/kg/d)......compound E12 (120 mg/kg/d). The mice were administrated for twice every day. After administration for 14 days, the mice were anesthetized with a 0.8% pentobarbital sodium solution (10 mL/kg) on Day 15; bronchoalveolar lavage fluids and lung tissues were taken for detecting the levels of hydroxyproline and TGF-β as golden standards for pulmonary fibrosis in the bronchoalveolar lavage fluids and the lung tissues. The pulmonary fibrosis resisting levels of the compounds in this patent application are evaluated. Results are shown in Table 2-5.

**Table 2-5 Detection results of hydroxyproline in bronchoalveolar lavage fluids and lung tissues**

| Group | Hydroxyproline in lung tissues (µg/mg) | Hydroxyproline in bronchoalveolar lavage fluids (µg/mg) | TGF-β in lung tissues (µg/L) | TGF-β in bronchoalveolar lavage fluids (µg/L) |
|---|---|---|---|---|
| Blank control group | 1.41 ± 0.14 | 0.71 ± 0.08 | 24.53 ± 2.14 | 4.06 ± 0.24 |
| Model group | 2.87 ± 0.18 | 1.57 ± 0.09 | 99.32 ± 8.26 | 9.32 ± 0.64 |
| Nintedanib positive control group | 2.36 ± 0.12 | 1.25 ± 0.14 | 75.32 ± 7.51 | 7.11 ± 0.24 |
| Hypoxanthine analogue A group | 2.69 ± 0.12 | 1.46 ± 0.13 | 96.47 ± 8.25 | 9.23 ± 0.26 |
| Hypoxanthine analogue B group | 2.97 ± 0.19 | 1.81 ± 0.14 | 91.53 ± 9.56 | 8.95 ± 0.68 |
| Hypoxanthine | 2.85 ± 0.25 | 1.12 ± 0.15 | 63.83 ± | 7.42 ± 1.4 |
| analogue C group | | | 7.52 | |
| Hypoxanthine analogue D group | 2.15 ± 0.35 | 1.94 ± 0.13 | 93.36 ± 8.51 | 6.42 ± 1.02 |
| Compound 1 | 1.45 ± 0.09 | 0.75 ± 0.04 | 24.15 ± 1.21 | 4.05 ± 0.15 |
| Compound 2 | 1.51 ± 0.08 | 0.74 ± 0.05 | 24.10 ± 2.14 | 4.02 ± 0.15 |
| Compound 3 | 1.31 ± 0.07 | 0.70 ± 0.05 | 24.15 ± 2.04 | 3.95 ± 0.19 |
| Compound 4 | 1.29 ± 0.08 | 0.71 ± 0.08 | 24.58 ± 1.95 | 3.95 ± 0.14 |
| Compound 5 | 1.43 ± 0.07 | 0.69 ± 0.09 | 24.18 ± 1.65 | 3.93 ± 0.18 |
| Compound 6 | 1.46 ± 0.07 | 0.68 ± 0.07 | 24.18 ± 2.14 | 3.91 ± 0.18 |
| Compound 7 | 1.42 ± 0.14 | 0.69 ± 0.08 | 25.15 ± 2.53 | 4.02 ± 0.15 |
| Compound 8 | 1.47 ± 0.10 | 0.72 ± 0.06 | 25.17 ± 2.35 | 4.06 ± 0.18 |
| Compound 9 | 1.41 ± 0.09 | 0.73 ± 0.09 | 25.65 ± 2.16 | 4.05 ± 0.14 |
| Compound 10 | 1.37 ± 0.08 | 0.74 ± 0.06 | 24.12 ± 2.25 | 4.21 ± 0.18 |
| Compound 11 | 1.43 ± 0.11 | 0.75 ± 0.05 | 23.98 ± 2.19 | 4.02 ± 0.15 |
| Compound 12 | 1.41 ± 0.12 | 0.76 ± 0.08 | 23.91 ± 2.38 | 4.06 ± 0.15 |
| Compound E-1 | 2.18 ± 0.32 | 1.14 ± 0.16 | 63.95 ± 2.19 | 6.51 ± 0.24 |
| Compound E-2 | 1.93 ± 0.24 | 1.16 ± 0.28 | 66.56 ± 1.95 | 6.21 ± 0.24 |
| Compound E-3 | 2.15 ± 0.23 | 1.03 ± 0.29 | 62.54 ± 1.28 | 6.35 ± 0.28 |
| Compound E-4 | 1.98 ± 0.44 | 1.14 ± 0.18 | 63.58 ± 2.95 | 6.84 ± 0.95 |
| Compound E-5 | 2.15 ± 0.35 | 1.17 ± 0.16 | 65.82 ± 3.54 | 6.51 ± 0.86 |
| Compound E-6 | 2.35 ± 0.24 | 1.18 ± 0.15 | 63.56 ± 6.54 | 6.14 ± 0.85 |
| Compound E-7 | 1.99 ± 0.25 | 1.15 ± 0.18 | 68.25 ± 2.58 | 6.38 ± 0.95 |
| Compound E-8 | 2.03 ± 0.51 | 1.06 ± 0.19 | 68.54 ± 2.25 | 6.51 ± 0.58 |
| Compound E-9 | 2.04 ± 0.15 | 1.05 ± 0.15 | 68.25 ± 6.23 | 6.81 ± 0.61 |
| Compound E-10 | 2.13 ± 0.18 | 1.04 ± 0.18 | 65.81 ± 5.28 | 6.75 ± 0.86 |
| Compound E-11 | 2.15 ± 0.21 | 1.08 ± 0.12 | 63.54 ± 4.56 | 6.91 ± 0.95 |
| Compound E-12 | 1.98 ± 0.21 | 1.07 ± 0.18 | 66.52 ± 5.21 | 6.35 ± 0.56 |

It can be seen from data in Table 2-5 that through data analysis via one-way ANOVA, the levels of hydroxyproline in the lung tissues of the mice in compound 1-12 intervention groups are all significantly inferior to those in model control group (*P*<0.001); the levels of hydroxyproline in the bronchoalveolar lavage fluids of the mice in compound 1-12 intervention groups are all significantly inferior to those in model control group (*P*<0.05); the levels of TGF-β in the lung tissues of the mice in compound 1-12 intervention groups are all significantly inferior to those in model control group (*P*<0.05); and the levels of TGF-β in the bronchoalveolar lavage fluids of the mice in compound 1-12 intervention groups are all significantly inferior to those in model control group (*P*<0.001). Importantly, it is further found that the levels of the hydroxyproline in the lung tissues of the mice in compound 1-12 intervention groups are all significantly inferior to those in hypoxanthine analogue A-D and compound E1-E12 control groups (from comparative statistic analysis on each group, *P* values are all smaller than 0.05, showing a significant difference); the levels of hydroxyproline in the bronchoalveolar lavage fluids of the mice in compound 1-12 intervention groups are all significantly inferior to those in hypoxanthine analogue A-D and compound E1-E12 control groups (from comparative statistic analysis on each group, *P* values are all smaller than 0.001, showing a significant difference); the levels of the TGF-β in the lung tissues of the mice in compound 1-12 intervention groups are all significantly inferior to those in hypoxanthine analogue A-D and compound E1-E12 control groups (from comparative statistic analysis on each group, *P* values are all smaller than 0.001, showing a significant difference); and the levels of the TGF-β in the bronchoalveolar lavage fluids of the mice in compound 1-12 intervention groups are all significantly inferior to those in hypoxanthine analogue A-D and compound E1-E12 control groups (from comparative statistic analysis on each group, *P* values are all smaller than 0.05, showing a significant difference). These results show that the compounds 1-12 prepared in example 1 have significant inhibition effects on the hypoxanthine and the TGF-β in the bronchoalveolar lavage fluids and the lung tissues of the pulmonary fibrosis model mice; and the pulmonary fibrosis resisting effects of the positive control Nintedanib, the hypoxanthine analogue A, the hydroxyproline B, the hypoxanthine analogue C, the hypoxanthine analogue D and the compounds E1-E12 are obviously inferior to those of the compounds 1-12. It can be obviously seen from Table 2-5 that the extent of the pulmonary fibrosis of the pulmonary fibrosis model mice is significantly improved under the actions of the compounds 1-12 prepared in example 1, which shows that the compounds 1-12 prepared in example 1 have significant pulmonary fibrosis resisting effects, and are superior to the Nintedanib, the hypoxanthine analogues A-D and the compounds E1-E12 in improvement effect.

### Example 3

### This example is used for detecting Streptococcus pneumoniae-induced pulmonary fibrosis resisting activities of compounds 1-12 prepared in example 1

Experimental method: an in-vivo *Streptococcus pneumoniae-induced* pulmonary fibrosis model was established: SPF class C57BL/6 mice (with weights being 22-25 g) were randomly and evenly divided into a plurality of groups, including blank control group, model group, Nintedanib positive control group, hypoxanthine analogue A group, hypoxanthine analogue B group, hypoxanthine analogue C group, compound 1 group, compound 2 group......compound 12 group, with 9 mice in each group, and were fed for 7 days in an SPF class animal center. On Day 1, the mice were lightly anesthetized by inhaling ethyl ether; the mice in blank control group were instilled with normal saline nasally; and the mice in other groups inhaled a 0.5 mL/kg *Streptococcus pneumoniae* liquid (with a concentration of 1.0×10⁹ CFU/mL) to lungs through tracheas by spontaneous breathing to induce pulmonary fibrosis. On Day 2, the mice received 5 mg/kg bleomycin through tracheas to induce their pulmonary fibrosis. After the model ws established for one week, the mice in each group were intragastrically administrated for intervention; the mice in blank control group and model group received the same weight and volume of normal saline as those in drug groups according to the weights; the mice in Nintedanib positive control group received a suitable volume of Nintedanib (120 mg/kg) according to the weights; the mice in hypoxanthine analogue A group, hypoxanthine analogue B group and hypoxanthine analogue C group respectively received hypoxanthine analogue A (120 mg/kg/d), hypoxanthine analogue B (120 mg/kg/d) and hypoxanthine analogue C (120 mg/kg/d); and the mice in compound 1 group, compound 2 group......compound 12 group respectively received compound 1 (120 mg/kg/d), compound 2 (120 mg/kg/d)......compound 12 (120 mg/kg/d) prepared in example 1. The mice were administrated for 3 times every day; after administration for 14 days, the mice were anesthetized with a 0.8% pentobarbital sodium solution (10 mL/kg) on Day 15; blood was taken from aorta abdominalis for detecting changes on blood routine and evaluating the severity of the pulmonary fibrosis of lung tissues; the paraffin sections of the lung tissues were subjected to HE staining and Ashcroft scoring. Results are shown in Table 3-1 and Table 3-2.

**Table 3-1 Table for inflammatory detection results of streptococcus pneumoniae-induced pulmonary fibrosis**

| Group | Leucocyte (10⁹/L) | Neutrophil (10⁹/L) | Lymphocyte (10⁹/L) |
|---|---|---|---|
| Blank control group | 6.43 | 1.45 | 5.97 |
| Model group | 143.22 | 94.07 | 99.02 |
| Nintedanib positive control group | 98.31 | 78.94 | 87.43 |
| Hypoxanthine analogue A group | 155.12 | 89.99 | 90.75 |
| Hypoxanthine analogue B group | 156.11 | 89.86 | 91.01 |
| Hypoxanthine analogue C group | 149.35 | 82.11 | 90.12 |
| Compound 1 | 5.52 | 2.00 | 5.99 |
| Compound 2 | 5.48 | 1.83 | 5.95 |
| Compound 3 | 5.62 | 1.60 | 5.77 |
| Compound 4 | 6.99 | 1.51 | 6.84 |
| Compound 5 | 6.75 | 1.82 | 7.64 |
| Compound 6 | 5.91 | 1.88 | 7.55 |
| Compound 7 | 6.39 | 1.88 | 7.72 |
| Compound 8 | 6.68 | 1.94 | 8.09 |
| Compound 9 | 6.73 | 1.89 | 7.11 |
| Compound 10 | 7.01 | 1.99 | 7.33 |
| Compound 11 | 6.29 | 1.81 | 6.21 |
| Compound 12 | 7.04 | 1.97 | 7.23 |

**Table 3-2 Ashcroft scoring table for extent of streptococcus pneumoniae-induced pulmonary fibrosis**

| Group | Ashcroft scores |
|---|---|
| Blank control group | 1.12 |
| Model group | 6.34 |
| Nintedanib positive control group | 4.55 |
| Hypoxanthine analogue A group | 6.12 |
| Hypoxanthine analogue B group | 6.14 |
| Hypoxanthine analogue C group | 6.21 |
| Compound 1 | 1.20 |
| Compound 2 | 1.09 |
| Compound 3 | 1.01 |
| Compound 4 | 1.29 |
| Compound 5 | 1.25 |
| Compound 6 | 1.27 |
| Compound 7 | 1.29 |
| Compound 8 | 1.31 |
| Compound 9 | 1.33 |
| Compound 10 | 1.38 |
| Compound 11 | 1.28 |
| Compound 12 | 1.39 |

From Table 3-1, it can be seen that the compounds 1-12 prepared in example 1 have significant inhibition effects on leucocytes, neutrophils and lymphocytes in blood of mice with *Streptococcus pneumoniae*-indocued pulmonary fibrosis; while the anti-inflammatory effects of the Nintedanib tablets, the hypoxanthine analogue A, the hypoxanthine analogue B and the hypoxanthine analogue C are obviously inferior to those of the compounds 1-12.

From Table 3-2, it can be obviously seen that the extent of the *Streptococcus pneumoniae*-induced pulmonary fibrosis is significantly improved under the actions of the compounds 1-12 prepared in example 1, that is, the extent of the pulmonary fibrosis is significantly reduced, which shows that the compounds 1-12 prepared in example 1 have significant *Streptococcus pneumoniae-induced* pulmonary fibrosis resisting effects and are superior to those of the Nintedanib tablets, the hypoxanthine analogue A, the hypoxanthine analogue B and the hypoxanthine analogue C in improvement effect.

### Example 4

### This example is used for detecting the influenza A virus-induced pulmonary fibrosis resisting activities of compounds 1-12 prepared in example 1.

Experimental method: an in-vivo influenza A virus-induced pulmonary fibrosis model was established: C57BL/6 mice (with weights being 22-25 g) were randomly and evenly divided into a plurality of groups, including blank control group, model group, Nintedanib positive control group, hypoxanthine analogue A group, hypoxanthine analogue B group, hypoxanthine analogue C group, compound 1 group, compound 2 group......compound 12 group, with 9 mice in each group. On Day 1, the mice in blank control group were instilled with normal saline nasally, and the mice in other groups were all infected with a FM1 strain of an influenza A (H1N1) virus 30 (µL) through nasal instillation. On Day 2, the mice received 5 mg/kg bleomycin through tracheas to induce their pulmonary fibrosis. After the model was established for two weeks, the mice in each group were intragastrically administrated for intervention for consecutive 14 days; the mice in blank control group and model group intragastrically received the same dosage of the normal saline as those in drug groups; the mice in Nintedanib positive control group received Nintedanib (120 mg/kg); the mice in hypoxanthine analogue A group, hypoxanthine analogue B group and hypoxanthine analogue C group respectively received hypoxanthine analogue A (120 mg/kg/d), hypoxanthine analogue B (120 mg/kg/d) and hypoxanthine analogue C (120 mg/kg/d); and the mice in compound 1 group, compound 2 group......compound 12 group respectively received compound 1 (120 mg/kg/d), compound 2 (120 mg/kg/d)......compound 12 (120 mg/kg/d) prepared in example 1. After administration for consecutive 14 days, the mice were observed every day, and their weights and death conditions were recorded. After administration, blood was taken from eyeballs for immediately detecting the expression levels of NF-κB, TNF-α, IL-1 and IL-6 in serum, and lung tissues were taken for HE detection and Ashcroft scoring. Results are shown in Table 4-1 and Table 4-2.

**Table 4-1 Detection result table for inflammatory indexes of influenza A virus-induced pulmonary fibrosis**

| Group | Serum indexes | | | |
|---|---|---|---|---|
| | NF-κB (pg/mL) | TNF-α (pg/mL) | IL-1β (pg/mL) | IL-6 (pg/mL) |
| Blank control group | 25.3 | 87.3 | 26.6 | 64.1 |
| Model group | 764.1 | 366.2 | 321.5 | 448.4 |
| Nintedanib positive control group | 625.2 | 243.6 | 220.7 | 349.1 |
| Hypoxanthine analogue A group | 780.3 | 339.4 | 323.1 | 450.9 |
| Hypoxanthine analogue B group | 771.9 | 341.6 | 329.7 | 422.7 |
| Hypoxanthine analogue C group | 782.1 | 333.1 | 338.1 | 450.0 |
| Compound 1 | 26.5 | 87.1 | 28.8 | 63.9 |
| Compound 2 | 25.5 | 87.3 | 27.1 | 62.7 |
| Compound 3 | 24.3 | 86.1 | 26.3 | 62.9 |
| Compound 4 | 27.4 | 89.8 | 29.6 | 65.1 |
| Compound 5 | 27.7 | 90.2 | 28.1 | 65.6 |
| Compound 6 | 27.8 | 90.3 | 28.5 | 66.1 |
| Compound 7 | 26.4 | 91.2 | 29.0 | 65.3 |
| Compound 8 | 26.8 | 89.9 | 28.6 | 66.0 |
| Compound 9 | 27.1 | 91.1 | 28.4 | 65.8 |
| Compound 10 | 27.7 | 89.6 | 29.3 | 64.9 |
| Compound 11 | 26.6 | 88.7 | 27.9 | 64.6 |
| Compound 12 | 28.1 | 90.8 | 29.9 | 64.7 |

**Table 4-2 Ashcroft scoring table for extent of influenza A virus-induced pulmonary fibrosis**

| Group | Ashcroft scores |
|---|---|
| Blank control group | 1.23 |
| Model group | 6.18 |
| Nintedanib positive control group | 4.27 |
| Hypoxanthine analogue A group | 6.11 |
| Hypoxanthine analogue B group | 6.07 |
| Hypoxanthine analogue C group | 6.09 |
| Compound 1 | 1.21 |
| Compound 2 | 1.25 |
| Compound 3 | 1.18 |
| Compound 4 | 1.29 |
| Compound 5 | 1.31 |
| Compound 6 | 1.26 |
| Compound 7 | 1.28 |
| Compound 8 | 1.28 |
| Compound 9 | 1.33 |
| Compound 10 | 1.30 |
| Compound 11 | 1.26 |
| Compound 12 | 1.31 |

From data in Table 4-1, it can be seen that the compounds 1-12 prepared in example 1 have the effects of significantly lowering the levels of IL-1β, IL-6, TNF-α and NF-κB in serum, which shows that the compounds 1-12 have significant influenza A virus-induced pulmonary fibrosis resisting activities, and are superior to hypoxanthine analogue A group, hypoxanthine analogue B group, hypoxanthine analogue C group and Nintedanib in effect. Also, it is found that there are no pulmonary fibrosis lesions in the lungs of the mice in compound 1-12 intervention groups.

From data in Table 4-2, it can be seen that the compounds 1-12 prepared in example 1 have the effects of significantly reducing the extent of the influenza A virus-induced pulmonary fibrosis model mice, which shows that the compounds 1-12 have significant improvement effects on the influenza A virus-induced pulmonary fibrosis, and are superior to hypoxanthine analogue A group, hypoxanthine analogue B group, hypoxanthine analogue C group and Nintedanib tablets in effect.

### Example 5

### This example is used for detecting influenza B virus-induced pulmonary fibrosis resisting activities of compounds 1-12 prepared in example 1.

Experimental method: an in-vivo influenza B virus-induced pulmonary fibrosis model was established: C57BL/6 mice (with weights being 22-25 g) were randomly and evenly divided into a plurality of groups, including blank control group, model group, Nintedanib positive control group, hypoxanthine analogue A group, hypoxanthine analogue B group, hypoxanthine analogue C group, compound 1 group, compound 2 group......compound 12 group, with 9 mice in each group. On Day 1, the mice in blank control group were instilled with normal saline nasally, and the mice in other groups were all infected with a strain of an influenza B (H7N9) virus (30 µL) through nasal instillation. On Day 2, the mice received 5 mg/kg bleomycin through tracheas to induce their pulmonary fibrosis. After the model was established for two weeks, the mice in each group were intragastrically administrated for intervention for consecutive 14 days; the mice in blank control group and model group intragastrically received the same dosage of normal saline as those in drug groups; the mice in Nintedanib positive control group received Nintedanib (120 mg/kg); the mice in hypoxanthine analogue A group, hypoxanthine analogue B group and hypoxanthine analogue C group respectively received hypoxanthine analogue A (120 mg/kg/d), hypoxanthine analogue B (120 mg/kg/d) and hypoxanthine analogue C (120 mg/kg/d); and the mice in compound 1 group, compound 2 group......compound 12 group respectively received compound 1 (120 mg/kg/d), compound 2 (120 mg/kg/d)......compound 12 (120 mg/kg/d) prepared in example 1. After administration for consecutive 14 days, the mice were observed every day, and their weights and death conditions were recorded. After administration, blood was taken from eyeballs for immediately detecting the expression levels of NF-κB, TNF-α, IL-1 and IL-6 in serum, and lung tissues were taken for HE detection and Ashcroft scoring. Results are shown in Table 5-1 and Table 5-2.

**Table 5-1 Detection result table for inflammatory indexes of influenza B virus-induced pulmonary fibrosis**

| Group | Serum indexes | | | |
|---|---|---|---|---|
| | NF-κB (pg/mL) | TNF-α (pg/mL) | IL-1β (pg/mL) | IL-6 (pg/mL) |
| Blank control group | 30.3 | 73.5 | 26.7 | 65.6 |
| Model group | 628.5 | 188.4 | 250.1 | 211.6 |
| Nintedanib positive control group | 529.7 | 141.3 | 166.3 | 176.1 |
| Hypoxanthine analogue A group | 630.5 | 191.3 | 239.9 | 194.8 |
| Hypoxanthine analogue B group | 627.1 | 190.2 | 240.6 | 193.1 |
| Hypoxanthine analogue C group | 632.4 | 198.9 | 241.9 | 194.9 |
| Compound 1 | 30.5 | 72.9 | 26.3 | 65.1 |
| Compound 2 | 29.5 | 72.6 | 26.6 | 65.4 |
| Compound 3 | 28.8 | 71.8 | 25.8 | 64.1 |
| Compound 4 | 29.3 | 73.9 | 27.6 | 66.7 |
| Compound 5 | 31.4 | 76.6 | 28.1 | 66.9 |
| Compound 6 | 32.4 | 75.3 | 27.2 | 65.6 |
| Compound 7 | 33.4 | 74.1 | 27.9 | 70.3 |
| Compound 8 | 31.9 | 73.5 | 27.1 | 66.5 |
| Compound 9 | 30.8 | 73.8 | 26.9 | 66.1 |
| Compound 10 | 31.2 | 74.7 | 27.8 | 65.5 |
| Compound 11 | 31.6 | 73.5 | 26.8 | 65.3 |
| Compound 12 | 32.9 | 75.4 | 27.8 | 69.7 |

**Table 5-2 Ashcroft scoring table for extent of influenza B virus-induced pulmonary fibrosis**

| Group | Ashcroft scores |
|---|---|
| Blank control group | 1.09 |
| Model group | 6.64 |
| Nintedanib positive control group | 4.08 |
| Hypoxanthine analogue A group | 6.26 |
| Hypoxanthine analogue B group | 6.21 |
| Hypoxanthine analogue C group | 6.15 |
| Compound 1 | 1.19 |
| Compound 2 | 1.09 |
| Compound 3 | 0.98 |
| Compound 4 | 1.17 |
| Compound 5 | 1.18 |
| Compound 6 | 1.14 |
| Compound 7 | 1.20 |
| Compound 8 | 1.16 |
| Compound 9 | 1.23 |
| Compound 10 | 1.25 |
| Compound 11 | 1.15 |
| Compound 12 | 1.26 |

From data in Table 5-1, it can be seen that the compounds 1-12 prepared in example 1 have the effects of significantly lowering the levels of IL-1β, IL-6, TNF-α and NF-κB in the serum of the mice with influenza B Virus-induced pulmonary fibrosis, which shows that the compounds 1-12 have significant influenza B virus-induced pulmonary fibrosis resisting activities, and are superior to the hypoxanthine analogue A, the hypoxanthine analogue B, the hypoxanthine analogue C and Nintedanib in effect. Also, it is found that there are no pulmonary fibrosis lesions in the lungs of the mice in compound 1-12 intervention groups.

From data in Table 5-2, it can be seen that the compounds 1-12 prepared in example 1 have the effects of significantly reducing the extent of the pulmonary fibrosis of the influenza B virus-induced pulmonary fibrosis model mice, which shows that the compounds 1-12 have significant improvement effects on influenza B virus-induced pulmonary fibrosis, and are superior to the hypoxanthine analogue A, the hypoxanthine analogue B, the hypoxanthine analogue C group and the Nintedanib tablets in effect.

### Example 6

### This example is used for detecting the coronavirus-induced pulmonary fibrosis resisting activities of compounds 1-12 prepared in example 1.

Experimental method: an in-vivo coronavirus-induced pulmonary fibrosis model mice was established: C57BL/6 mice (with weights being 22-25 g) were randomly and evenly divided into a plurality of groups, including blank control group, model group, Nintedanib positive control group, hypoxanthine analogue A group, hypoxanthine analogue B group, hypoxanthine analogue C group, compound 1 group, compound 2 group......compound 12 group, with 9 mice in each group. On Day 1, the mice in the blank control group were instilled with normal saline nasally, and the mice in other groups were all infected with HcoV-OC43 coronavirus strains (30 µL) through nasal instillation. On Day 2, the mice received 5 mg/kg bleomycin through tracheas to induce their pulmonary fibrosis. After the model was established for two weeks, the mice in each group were intragastrically administrated for intervention for consecutive 14 days; the mice in blank control group and model group intragastrically received the same dosage of the normal saline as those in drug groups; the mice in Nintedanib positive control group received Nintedanib (120 mg/kg); the mice in hypoxanthine analogue A group, hypoxanthine analogue B group and hypoxanthine analogue C group respectively received hypoxanthine analogue A (120 mg/kg/d), hypoxanthine analogue B (120 mg/kg/d) and hypoxanthine analogue C (120 mg/kg/d); and the mice in compound 1 group, compound 2 group......compound 12 group respectively received compound 1 (120 mg/kg/d), compound 2 (120 mg/kg/d)......compound 12 (120 mg/kg/d) prepared in example 1. After administration for consecutive 14 days, the mice were observed every day, and their weights and death conditions were recorded. After administration, blood was taken from eyeballs for immediately detecting the expression levels of NF-κB, TNF-α, IL-1 and IL-6 in serum, and lung tissues were taken for HE detection and Ashcroft scoring. Results are shown in Table 6-1 and Table 6-2.

**Table 6-1 Detection result table for inflammatory indexes of coronavirus-induced pulmonary fibrosis**

| Group | Serum indexes | | | |
|---|---|---|---|---|
| | NF-κB (pg/mL) | TNF-α (pg/mL) | IL-1β (pg/mL) | IL-6 (pg/mL) |
| Blank control group | 25.8 | 51.8 | 32.2 | 63.5 |
| Model group | 731.6 | 457.2 | 679.1 | 515.7 |
| Nintedanib positive control group | 521.3 | 411.3 | 525.3 | 421.5 |
| Hypoxanthine analogue A group | 719.1 | 450.7 | 688.8 | 519.4 |
| Hypoxanthine analogue B group | 730.1 | 454.4 | 560.6 | 522.2 |
| Hypoxanthine analogue C group | 729.6 | 449.1 | 530.1 | 530.9 |
| Compound 1 | 23.9 | 51.8 | 31.8 | 62.7 |
| Compound 2 | 24.6 | 51.2 | 32.0 | 62.6 |
| Compound 3 | 23.7 | 49.9 | 31.3 | 61.7 |
| Compound 4 | 26.7 | 52.9 | 35.5 | 63.9 |
| Compound 5 | 25.9 | 52.6 | 37.1 | 64.9 |
| Compound 6 | 29.3 | 56.2 | 39.5 | 65.7 |
| Compound 7 | 25.6 | 55.9 | 33.2 | 64.3 |
| Compound 8 | 28.3 | 56.1 | 36.6 | 66.7 |
| Compound 9 | 24.4 | 55.1 | 35.9 | 64.3 |
| Compound 10 | 25.3 | 56.2 | 37.7 | 68.2 |
| Compound 11 | 25.1 | 52.1 | 35.4 | 63.3 |
| Compound 12 | 27.7 | 54.4 | 35.8 | 66.5 |

**Table 6-2 Ashcroft scoring table for extent of coronavirus-induced pulmonary fibrosis**

| Group | Ashcroft scores |
|---|---|
| Blank control group | 1.28 |
| Model group | 6.43 |
| Nintedanib positive control group | 4.11 |
| Hypoxanthine analogue A group | 6.23 |
| Hypoxanthine analogue B group | 6.12 |
| Hypoxanthine analogue C group | 6.31 |
| Compound 1 | 1.18 |
| Compound 2 | 1.16 |
| Compound 3 | 1.03 |
| Compound 4 | 1.39 |
| Compound 5 | 1.31 |
| Compound 6 | 1.30 |
| Compound 7 | 1.49 |
| Compound 8 | 1.29 |
| Compound 9 | 1.31 |
| Compound 10 | 1.41 |
| Compound 11 | 1.29 |
| Compound 12 | 1.37 |

From Table 6-1, it can be seen that the compounds 1-12 prepared in example 1 all significantly lower the levels of NF-κB, TNF-α, IL-1β and IL-6 in serum, which shows that the compounds 1-12 have significant coronavirus (HcoV-OC43)-induced pulmonary fibrosis resisting activities, and are superior to Nintedanib as well as the hypoxanthine analogue A, the hypoxanthine analogue B and the hypoxanthine analogue C in effect. Also, it is found that there are no pulmonary fibrosis lesions in the lungs of the mice in compound 1-12 intervention groups.

From Table 6-2, it can be seen that the compounds 1-12 prepared in example 1 all significantly lower the extent of the pulmonary fibrosis of the coronavirus-induced pulmonary fibrosis model mice, which shows that the compounds 1-12 have significant coronavirus-induced pulmonary fibrosis resisting effects, and are superior to Nintedanib as well as the hypoxanthine analogue A, the hypoxanthine analogue B and the hypoxanthine analogue C in effect.

### Example 7

### This example is used for detecting the novel coronavirus-induced pulmonary fibrosis resisting activities of compounds 1-12 prepared in example 1.

Experimental method: an in-vivo novel coronavirus-induced pulmonary fibrosis model was established: C57BL/6 mice (with weights being 22-25 g) were randomly and evenly divided into a plurality of groups, including blank control group, model group, Nintedanib positive control group, hypoxanthine analogue A group, hypoxanthine analogue B group, hypoxanthine analogue C group, compound 1 group, a compound 2 group......compound 12 group, with 9 mice in each group. On Day 1, the mice in blank control group were instilled with normal saline nasally, and the mice in other groups were all infected with COVID-19 novel coronavirus strains (30 µL) through nasal instillation. On Day 2, the mice received 5 mg/kg bleomycin through tracheas to induce their pulmonary fibrosis. After the model was established for two weeks, the mice in each group were intragastrically administrated for intervention for consecutive 14 days; the mice in blank control group and model group intragastrically received the same dosage of normal saline as those in drug groups; the mice in Nintedanib positive control group received Nintedanib (120 mg/kg); the mice in hypoxanthine analogue A group, hypoxanthine analogue B group and hypoxanthine analogue C group respectively received hypoxanthine analogue A (120 mg/kg/d), hypoxanthine analogue B (120 mg/kg/d) and hypoxanthine analogue C (120 mg/kg/d); and the mice in compound 1 group, compound 2 group......compound 12 group respectively received compound 1 (120 mg/kg/d), compound 2 (120 mg/kg/d)......compound 12 (120 mg/kg/d) prepared in example 1. After administration for consecutive 14 days, the mice were observed every day, and their weights and death conditions were recorded. After administration, blood was taken from eyeballs for immediately detecting the expression levels of NF-κB, TNF-α, IL-1 and IL-6 in serum, and lung tissues were taken for HE detection and Ashcroft scoring. Results are shown in Table 7-1 and Table 7-2.

**Table 7-1 Detection result table for inflammatory indexes of novel coronavirus-induced pulmonary fibrosis**

| Group | Serum indexes | | | |
|---|---|---|---|---|
| | NF-κB (pg/mL) | TNF-α (pg/mL) | IL-1β (pg/mL) | IL-6 (pg/mL) |
| Blank control group | 25.7 | 69.3 | 36.1 | 66.2 |
| Model group | 730.8 | 538.6 | 560.4 | 531.6 |
| Nintedanib positive control group | 732.7 | 539.8 | 561.2 | 523.7 |
| Hypoxanthine analogue A group | 731.3 | 400.5 | 489.0 | 554.5 |
| Hypoxanthine analogue B group | 741.1 | 401.9 | 491.8 | 552.3 |
| Hypoxanthine analogue C group | 739.2 | 542.4 | 572.7 | 543.7 |
| Compound 1 | 26.6 | 63.3 | 36.4 | 68.3 |
| Compound 2 | 23.5 | 64.8 | 35.1 | 66.0 |
| Compound 3 | 24.7 | 63.5 | 36.3 | 65.3 |
| Compound 4 | 25.2 | 65.9 | 37.2 | 64.5 |
| Compound 5 | 24.3 | 66.1 | 37.5 | 66.4 |
| Compound 6 | 21.2 | 68.3 | 38.7 | 67.1 |
| Compound 7 | 24.6 | 67.5 | 39.9 | 66.4 |
| Compound 8 | 23.4 | 66.7 | 38.4 | 68.2 |
| Compound 9 | 22.9 | 68.8 | 39.6 | 69.1 |
| Compound 10 | 26.5 | 68.9 | 38.1 | 67.5 |
| Compound 11 | 26.7 | 69.0 | 37.2 | 63.3 |
| Compound 12 | 27.3 | 68.4 | 38.9 | 66.8 |

**Table 7-2 Ashcroft scoring table for extent of novel coronavirus-induced pulmonary fibrosis**

| Group | Ashcroft scores |
|---|---|
| Blank control group | 1.28 |
| Model group | 6.35 |
| Nintedanib positive control group | 6.04 |
| Hypoxanthine analogue A group | 6.13 |
| Hypoxanthine analogue B group | 6.38 |
| Hypoxanthine analogue C group | 6.71 |
| Compound 1 | 1.08 |
| Compound 2 | 1.12 |
| Compound 3 | 1.19 |
| Compound 4 | 1.50 |
| Compound 5 | 1.11 |
| Compound 6 | 1.34 |
| Compound 7 | 1.15 |
| Compound 8 | 1.24 |
| Compound 9 | 1.34 |
| Compound 10 | 1.67 |
| Compound 11 | 1.31 |
| Compound 12 | 1.18 |

From Table 7-1, it can be seen that the compounds 1-12 prepared in example 1 all significantly lower the levels of NF-κB, TNF-α, IL-1β and IL-6 in serum, which shows that the compounds 1-12 have significant novel coronavirus-induced pulmonary fibrosis resisting activities, and are superior to Nintedanib as well as the hypoxanthine analogue A, the hypoxanthine analogue B and the hypoxanthine analogue C in effect. Also, it is found that there are no pulmonary fibrosis lesions in the lungs of the mice in compound 1-12 intervention groups.

From Table 7-2, it can be seen that the compounds 1-12 prepared in example 1 all significantly lower the extent of the pulmonary fibrosis of novel coronavirus-induced pulmonary fibrosis model mice, which shows that the compounds 1-12 have significant pulmonary novel coronavirus-induced fibrosis resisting effects, and are superior to Nintedanib as well as the hypoxanthine analogue A, the hypoxanthine analogue B and the hypoxanthine analogue C in effect.

### Example 8

### This example is used for detecting the mycoplasmas-induced pulmonary fibrosis resisting activities of compounds 1-12 prepared in example 1.

Experimental method: an in-vivo mycoplasma-induced pulmonary fibrosis model was established: C57BL/6 mice (with weights being 22-25 g) were randomly and evenly divided into a plurality of groups, including blank control group, model group, Nintedanib positive control group, hypoxanthine analogue A group, hypoxanthine analogue B group, hypoxanthine analogue C group, compound 1 group, compound 2 group......compound 12 group, with 9 mice in each group. Before a model was established, the mice were anesthetized with diethyl ether. In addition to that the mice in blank control group with instilled with 100 µL of normal saline nasally, the mice in other groups with slowly instilled with an equal volume of a MPFH strain solution (containing 1×10⁷ mL⁻¹ MPFH strain) to the nasal cavity, for consecutive 3 days, and inhaled the MPFH strain solution into bronchi. After instillation with mycoplasmas through tracheas, the mice received 5 mg/kg bleomycin through tracheas to induce their pulmonary fibrosis. After the model was established for two weeks, the mice in each group were intragastrically administrated for intervention for consecutive 14 days; the mice in blank control group and model group intragastrically received an equal dosage of normal saline as those in drug groups; the mice in Nintedanib positive control group received Nintedanib (120 mg/kg); the mice in hypoxanthine analogue A group, hypoxanthine analogue B group and hypoxanthine analogue C group respectively received hypoxanthine analogue A (120 mg/kg/d), hypoxanthine analogue B (120 mg/kg/d) and hypoxanthine analogue C (120 mg/kg/d); and the mice in compound 1 group, compound 2 group......compound 12 group respectively received compound 1 (120 mg/kg/d), compound 2 (120 mg/kg/d)......compound 12 (120 mg/kg/d) prepared in example 1. The mice were administrated once a day to be treated for consecutive 14 days. The mice were observed every day, and their weights and death conditions were recorded. On the last day of administration, the mice were sacrificed; and blood was taken from eyeballs and kept at -80°C for detecting blood routine indexes. Meanwhile, lungs were lavaged with normal saline and separated to collect lavage fluids for detecting counts and classes of leucocytes; and lung tissues were taken for HE straining and Ashcroft scoring on the extent of pulmonary fibrosis. Results are shown in Table 8-1 and Table 8-2.

**Table 8-1 Table for inflammatory detection results of mycoplasmas-induced pulmonary fibrosis**

| Group | Leucocyte (10⁹/L) | Neutrophil (10⁹/L) | Lymphocyte (10⁹/L) |
|---|---|---|---|
| Blank control group | 6.56 | 22.90 | 73.33 |
| Model group | 113.98 | 233.15 | 564.09 |
| Nintedanib positive control group | 122.15 | 232.24 | 565.69 |
| Hypoxanthine analogue A group | 116.79 | 234.53 | 567.15 |
| Hypoxanthine analogue B group | 118.58 | 233.92 | 568.05 |
| Hypoxanthine analogue C group | 120.98 | 238.07 | 571.34 |
| Compound 1 | 7.01 | 21.01 | 72.56 |
| Compound 2 | 6.42 | 21.93 | 72.69 |
| Compound 3 | 6.83 | 22.12 | 72.33 |
| Compound 4 | 6.79 | 22.54 | 73.37 |
| Compound 5 | 6.86 | 21.91 | 74.39 |
| Compound 6 | 6.05 | 22.72 | 71.57 |
| Compound 7 | 7.15 | 21.45 | 73.29 |
| Compound 8 | 7.26 | 23.62 | 72.52 |
| Compound 9 | 6.79 | 22.06 | 73.19 |
| Compound 10 | 6.18 | 21.11 | 72.54 |
| Compound 11 | 6.70 | 22.47 | 73.25 |
| Compound 12 | 6.12 | 21.68 | 74.46 |

**Table 8-2 Ashcroft scoring table for extent of mycoplasmas-induced pulmonary fibrosis**

| Group | Ashcroft scores |
|---|---|
| Blank control group | 1.21 |
| Model group | 6.63 |
| Nintedanib positive control group | 5.45 |
| Hypoxanthine analogue A group | 6.64 |
| Hypoxanthine analogue B group | 6.30 |
| Hypoxanthine analogue C group | 6.41 |
| Compound 1 | 1.12 |
| Compound 2 | 1.20 |
| Compound 3 | 0.96 |
| Compound 4 | 1.05 |
| Compound 5 | 1.23 |
| Compound 6 | 1.17 |
| Compound 7 | 1.25 |
| Compound 8 | 1.26 |
| Compound 9 | 1.38 |
| Compound 10 | 1.45 |
| Compound 11 | 1.50 |
| Compound 12 | 1.12 |

From data in Table 8-1, it can be seen that the compounds 1-12 prepared in example 1 have the effects of significantly lowering the levels of neutrophils, lymphocytes and leucocytes in blood of mice infected with mycoplasmas, which shows that the compounds 1-12 have mycoplasmas infection resisting effects and pulmonary fibrosis resisting activities, and are superior to the hypoxanthine analogue A, the hypoxanthine analogue B, the hypoxanthine analogue C and Nintedanib in effect. Also, it is found that there are no pulmonary fibrosis lesions in the lungs of the mice in compound 1-12 intervention groups.

From data in Table 8-2, it can be seen that the compounds 1-12 prepared in example 1 have the effects of significantly reducing the extent of the pulmonary fibrosis of the mycoplasma-induced pulmonary fibrosis model mice, which shows that the compounds 1-12 have significant mycoplasmas-induced pulmonary fibrosis improvement effects, and are superior to the hypoxanthine analogue A, the hypoxanthine analogue B, the hypoxanthine analogue C and the Nintedanib tablets in effect.

The above descriptions are merely specific embodiments of the present invention, but the protective scope of the present invention is not limited thereto. Any change or substitution that can be easily conceived by any technician familiar with the technical field within the technical scope disclosed by the present invention should be covered within the protective scope of the present invention. Therefore, the protective scope of the present invention should be defined only in accordance with the appended claims.

## Claims

1. Use of a hypoxanthine compound in preparing a drug for treating pulmonary fibrosis, wherein the hypoxanthine compound has the activity of treating the pulmonary fibrosis, and the hypoxanthine compound has one of the following structures: 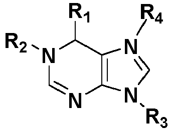 wherein:
R₁ is optionally O, N, C, S or =O;
R₂, R₃ and R₄ are optionally H, C₁-C₁₈ alkyl, C₁-C₁₈ alkyl substituted with a halogen, trifluoromethyl, sulfonyl, sulfonamide, sulfinyl, an amino acid group, 2-[bis[(pivaloyloxy)methoxy]phosphinyl]methoxy]ethyl, a C₁-C₁₈ fatty acid group, C₃-C₁₂ heterocyclyl, and C₁-C₁₈ fatty acid; or R₂, R₃ and R₄ are optionally C₁-C₁₈ alkyl or fatty acid group substituted with an oxygen atom, a sulphur atom or a nitrogen atom; and R₃ and R₄ are attached to a non-substituted position N via double bonds when being monosubstituted, and have no double bonds when being fully substituted.

2. The use of the hypoxanthine compound in preparing the drug for treating the pulmonary fibrosis according to claim 1, wherein the hypoxanthine compound is one or more of the following compounds:

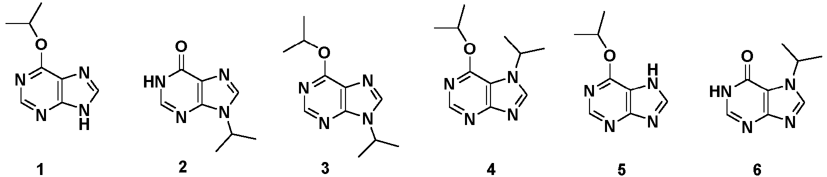

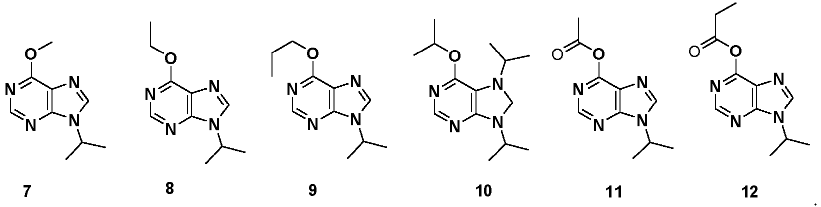

3. The use of the hypoxanthine compound in preparing the drug for treating the pulmonary fibrosis according to claim 1, wherein the drug for treating the pulmonary fibrosis comprises a drug having the efficacy of preventing and treating pulmonary fibrosis and complications thereof.

4. The use of the hypoxanthine compound in preparing the drug for treating the pulmonary fibrosis according to claim 1, wherein the drug for treating the pulmonary fibrosis is a preparation prepared by using the hypoxanthine compound or a salt thereof as an active ingredient in combination with pharmaceutically acceptable excipient or auxiliary component.

5. The use of the hypoxanthine compound in preparing the drug for treating the pulmonary fibrosis according to claim 4, wherein the preparation is an oral preparation, an injection preparation or a nasal mucosal administration preparation.

6. The use of the hypoxanthine compound in preparing the drug for treating the pulmonary fibrosis according to claim 1, wherein the pulmonary fibrosis comprises one or more of primary pulmonary fibrosis, secondary pulmonary fibrosis, idiopathic pulmonary fibrosis, pulmonary interstitial fibrosis, and interstitial pneumonia.

7. The use of the hypoxanthine compound in preparing the drug for treating the pulmonary fibrosis according to claim 6, wherein the pulmonary fibrosis comprises one or more of bacterial pulmonary fibrosis, viral pulmonary fibrosis, mycoplasma pulmonary fibrosis, chlamydia pulmonary fibrosis, immunological pulmonary fibrosis, and fungus pulmonary fibrosis.

8. The use of the hypoxanthine compound in preparing the drug for treating the pulmonary fibrosis according to claim 7, wherein the pulmonary fibrosis comprises one or more of *Streptococcus pneumoniae*-induced pulmonary fibrosis, influenza A virus-induced pulmonary fibrosis, influenza B virus-induced pulmonary fibrosis, coronavirus-induced pulmonary fibrosis, and novel coronavirus-induced pulmonary fibrosis.

9. The use of the hypoxanthine compound in preparing the drug for treating the pulmonary fibrosis according to claim 8, wherein the pulmonary fibrosis further comprises one or more of *Klebsiella pneumonia*-induced pulmonary fibrosis, *Streptococcus pneumoniae-induced* pulmonary fibrosis, vancomycin-resistant *Enterococcus*-induced pulmonary fibrosis, drug-resistant *Staphylococcus aureus*-induced pulmonary fibrosis, and *Acinetobacter baumannii*-induced pulmonary fibrosis.

10. A compound having the following structure: 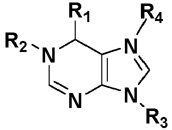 wherein:
R₁ is optionally O, N, C, S or =O;
R₂, R₃ and R₄ are optionally H, C₁-C₁₈ alkyl, C₁-C₁₈ alkyl substituted with a halogen, trifluoromethyl, sulfonyl, sulfonamide, sulfinyl, an amino acid group, 2-[bis[(pivaloyloxy)methoxy]phosphinyl]methoxy]ethyl, a C₁-C₁₈ fatty acid group, C₃-C₁₂ heterocyclyl, and C₁-C₁₈ fatty acid; or R₂, R₃ and R₄ are optionally C₁-C₁₈ alkyl or fatty acid group substituted with an oxygen atom, a sulphur atom or a nitrogen atom; and R₃ and R₄ are attached to a non-substituted position N via double bonds when being monosubstituted, and have no double bonds when being fully substituted.

11. The compound according to claim 10, wherein the compound is selected from the following group:

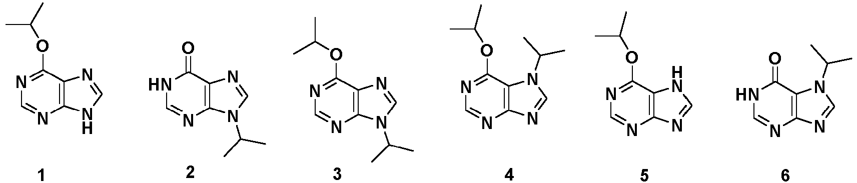

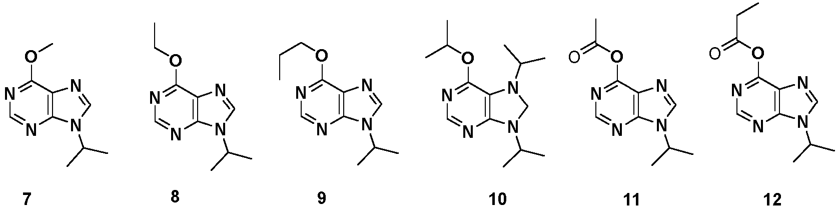

12. A pharmaceutical composition comprising the compound according to claim 10 or 11 or the pharmaceutically acceptable salt thereof.

13. The pharmaceutical composition according to claim 12, further comprising pharmaceutically acceptable excipient or auxiliary component.

14. The pharmaceutical composition according to claim 12 or 13, wherein the pharmaceutical composition is an oral preparation, an injection preparation or a nasal mucosal administration preparation.

15. A method of treating pulmonary fibrosis, comprising: administrating an effective amount of the compound according to claim 10 or 11 or the pharmaceutically acceptable salt thereof, or the pharmaceutical composition according to any of claims 12 to 14 to a subject in need thereof.

16. The method according to claim 15, wherein the pulmonary fibrosis comprises one or more of primary pulmonary fibrosis, secondary pulmonary fibrosis, idiopathic pulmonary fibrosis, pulmonary interstitial fibrosis, and interstitial pneumonia.

17. The method according to claim 15, wherein the pulmonary fibrosis comprises one or more of bacterial pulmonary fibrosis, viral pulmonary fibrosis, mycoplasma pulmonary fibrosis, chlamydia pulmonary fibrosis, immunological pulmonary fibrosis, and fungus pulmonary fibrosis.

18. The method according to claim 15, wherein the pulmonary fibrosis comprises one or more of *Streptococcus pneumoniae-induced* pulmonary fibrosis, influenza A virus-induced pulmonary fibrosis, influenza B virus-induced pulmonary fibrosis, coronavirus-induced pulmonary fibrosis, and novel coronavirus-induced pulmonary fibrosis.

19. The method according to claim 15, wherein the pulmonary fibrosis comprises one or more of pulmonary fibrosis caused by *Klebsiella pneumonia*-induced pulmonary fibrosis, *Streptococcus pneumoniae*-induced pulmonary fibrosis, vancomycin-resistant *Enterococcus*-induced pulmonary fibrosis, drug-resistant *Staphylococcus aureus*-induced pulmonary fibrosis, and *Acinetobacter baumannii*-induced pulmonary fibrosis.
